# EUROPEAN PATENT APPLICATION

(11) **EP 3 967 337 A1**
(43) Date of publication of application: **16.03.2022**
(21) Application number: 20806002.0
(22) Date of filing: 04.03.2020
(51) Int. Cl.: A61M 5/14, A61M 5/158, A61M 25/06

(54) **INJECTION TOOL FOR ENDOSCOPE**

(30) Priority: 10.05.2019 JP 2019089562
(71) Applicant: LAKE.E2 Co., Ltd., Okaya-shi, Nagano 394-0028 (JP)
(72) Inventor: NISHIMURA Miyuki, Okaya-shi Nagano 394-0028 (JP)
(74) Representative: Schweiger, Martin
(86) International application number: PCT/JP2020/009107
(87) International publication number: WO 2020/230415

(57) **Abstract**

An endoscope injection tool includes a needle body in a distal end of an elongated hollow inner tube, an elongated hollow outer tube allowing the inner tube to be insert longitudinally, an operation unit having an operation unit body attached to the proximal end of the outer tube and slider inserted in lumen of the operation unit body and moved longitudinally with the inner tube, and an inner guide, the outer tube has an expanded stopper in a base end for locking to a reduced diameter portion of the operation unit body when inserted from the proximal end of the operation unit body, the inner guide has a contact portion bringing the stopper of the outer tube into contact with the reduced diameter portion of the operation unit body so as to fix. The outer tube can be easily assembled and the needle body's protruded state can be easily determined.

## Description

### TECHNICAL FIELD

The present invention relates to an injection tool for an endoscope which is inserted into a body cavity for injecting fluid to a submucosal layer within a body cavity.

### BACKGROUND ART

In general, when performing an endoscopic mucosal resection (EMR) and an endoscopic submucosal dissection (ESD) removing a mucous membrane such as an esophagus, a stomach and a large intestine, a submucosal layer is kept afloat by injecting a normal saline or a drug solution (hereinafter, refer simply to as fluid) to the submucosal layer with an injection tool for an endoscope, and is removed by a high frequency knife or a high frequency snare.

The injection tool for the endoscope is configured to tap a needle body in a distal end thereof to an affected area so as to inject fluid in a state in which the injection tool for the endoscope is inserted into a channel of the endoscope and protrudes out of a distal end of the channel of the endoscope, has an inner tube which is provided with the needle body in a distal end thereof and an outer tube which allows the inner tube to freely insert thereto, and can project and set the needle body with respect to the outer tube with an operation of an operation unit provided in the proximal end side. The fluid is injected to the affected area via the inner tube and the needle body from a syringe barrel installed to the operation unit in a state in which the needle body protrudes out of the outer tube.

The operation unit of the injection tool for the endoscope is provided with an operation unit body coupled to the outer tube, and a slider coupled to the inner tube, and is configured to allow the needle body to project and set with respect to the distal end of the outer tube by operating the slider to move forward and backward with respect to the operation unit body. For example, the operation units described in the following patent literatures can be listed up.

In an injection tool for an endoscope described in patent literature 1, an inner peripheral surface of an operation unit body is tapered toward a distal end, and an outer peripheral surface of a slider is tapered. Thus, both the tapered surfaces can be fixed by friction in an aligned state when the slider is pressed into the operation unit body, thereby keeping a protruded state of a needle body.

In an injection tool for an endoscope described in patent literature 2, an operation unit body is provided with a longitudinal groove and hook-like lock grooves are provided in a distal end and a proximal end of the longitudinal groove. Thus, a projection provided in the slider is slid along the groove and the projection of the slider is engaged with the lock grooves, thereby fixing in a protruded state or a stored state of the needle body.

### CITATION LIST

### PATENT LITERATURE

Patent Literature 1: Japanese Patent Unexamined Publication No. H02-139649
Patent Literature 2: Japanese Utility Model Unexamined Publication No. H01-68052

### SUMMARY OF THE INVENTION

### TECHNICAL PROBLEM

However, the injection tool for the endoscope described in the above-described patent literature 1 is configured to fix on the basis of the friction between the inner peripheral surface of the operation unit and the outer peripheral surface of the slider. Accordingly, there is a problem that the protrusion of the needle body may be insufficient owing to a strength of the operator's force, and a position to be fixed is difficult to be known. Further, in the injection tool for the endoscope described in the patent literature 2, an operation for rotating the slider is required when moving the projection of the slider to the lock groove from the longitudinal groove. Accordingly, it has a problem that the operation can not be smoothly performed.

Further, in the injection tools for the endoscope of the above-described patent literatures 1 and 2, any specific method for attaching the outer tube to the operation unit body is not disclosed. However, in general, the attachment is frequently achieved by using an adhesive agent. Thus, this case has a problem of a lack of safety caused by deterioration of the adhesive agent.

Taking into consideration the problems mentioned above, an object of the present invention is to provide an injection tool for an endoscope in which a protruded state of a needle body can be simply kept, an operator can easily know that a slider is arranged at a fixed position (that the needle body is in the protruded state), and the injection tool can be easily assembled and is safe.

### SOLUTION TO PROBLEM

In order to achieve the object, according to a first aspect of the present invention, there is provided an injection tool for an endoscope including an elongated hollow inner tube for allowing the fluid to flow into, a hollow needle body provided in a distal end side of the inner tube, an elongated hollow outer tube into which the inner tube is insertable to be movable forward and backward in a longitudinal direction, an operation unit having an elongated hollow operation unit body with a lumen attached to a proximal end side of the outer tube and passing through the inner tube, and a slider having the inner tube attached thereto, inserted into the lumen of the operation unit body and moved forward and backward in the longitudinal direction, and an inner guide inserted from the proximal end side of the operation unit body, and allowing the needle body to project and set with respect to the distal end of the outer tube by operating the slider of the operation unit to move forward and backward in the longitudinal direction, wherein the slider has in a distal end thereof a first projection protruding in an outer peripheral direction with an elastic force, the operation unit body has a first hole open to the distal end side in such a manner that the first projection in the distal end of the slider fits thereto when the needle body in in the protruded state from the outer tube, the outer tube has in a proximal end thereof a stopper expanded to an outer peripheral direction in such a manner as to lock to a reduced diameter portion of the operation unit body when the outer tube is inserted from the proximal end side of the operation unit body, and the inner guide has a contact portion bringing the stopper of the outer tube into contact with the reduced diameter portion of the operation unit body so as to fix.

Further, according to a second aspect of the present invention, the first projection is formed into a mountain shape which is fitted with a slope to the first hole opened to the operation unit body. According to a third aspect of the present invention, the slider has a second projection protruding toward the outer peripheral direction, and the operation unit body has at a symmetrical position of the first hole in the outer peripheral direction a guide groove to which the second projection is fitted to allow the operation unit body to move at a predetermined distance in the longitudinal direction.

Further, according to a fourth aspect of the present invention, the slider has a first arm portion having in a distal end thereof the first projection, and a second arm portion having in a distal end thereof the second projection. According to a fifth aspect of the present invention, the inner guide has in the proximal end thereof a protruding piece protruding in the outer peripheral direction, and the operation unit body has closer to the distal end side than the first hole a second hole to which the protruding piece fits.

Further, according to a sixth aspect of the present invention, the slider has a rectangular parallelepiped rib having a long side in a longitudinal direction for guiding an insertion direction of the slider, and the operation unit body has in a longitudinal direction a rib guide groove to which the rib is fitted.

### EFFECT OF INVENTION

In the injection tool for the endoscope according to the present invention, the operator can easily know that the slider is arranged at the fixed position (that the needle body is in the protruded state) on the basis of the structure mentioned above, and the outer tube can be easily attached without using any adhesive agent.

### BRIEF DESCRIPTION OF DRAWINGS

Fig. 1A is a plan view showing an exploded and assembled state of an injection tool for an endoscope according to an embodiment of the present invention.
Figs. 1B and 1C are plan views showing a total appearance of the injection tool for the endoscope according to the present embodiment, in which Fig. 1B is a view showing a stored state of a needle body 3, and Fig. 1C is a view showing a protruded state of the needle body 3.
Figs. 2A and 2B are views showing a cross section in a state in which the injection tool for the endoscope is rotated at 45 degrees, in which Fig. 2A is a view showing the stored state of the needle body 3, and Fig. 2B is a view showing the protruded state of the needle body 3.
Figs. 3A to 3E are views for describing an operation unit body according to the present invention, in which Fig. 3A is a top elevational view, Fig. 3B is a side elevational view, Fig. 3C is a bottom elevational view, Fig. 3D is a view as seen from a distal end direction, and Fig. 3E is a view as seen from a proximal end direction.
Figs. 4A to 4E are views for describing a slider according to the present invention, in which Fig. 4A is a top elevational view, Fig. 4B is a side elevational view, Fig. 4C is a bottom elevational view, Fig. 4D is a view as seen from a distal end direction, and Fig. 4C is a view as seen from a proximal end direction.
Figs. 5A to 5D are views for describing an inner guide according to the present invention, in which Fig. 5A is a top elevational view, Fig. 5B is a side elevational view, Fig. 5C is a view as seen from a distal end direction, and Fig. 5D is a view as seen from a proximal end direction.

### DESCRIPTION OF EMBODIMENTS

A description will be in detail given below of embodiments of a treatment tool for an endoscope according to the present invention with reference to the accompanying drawings.

### [First Embodiment]

### [Overall structure]

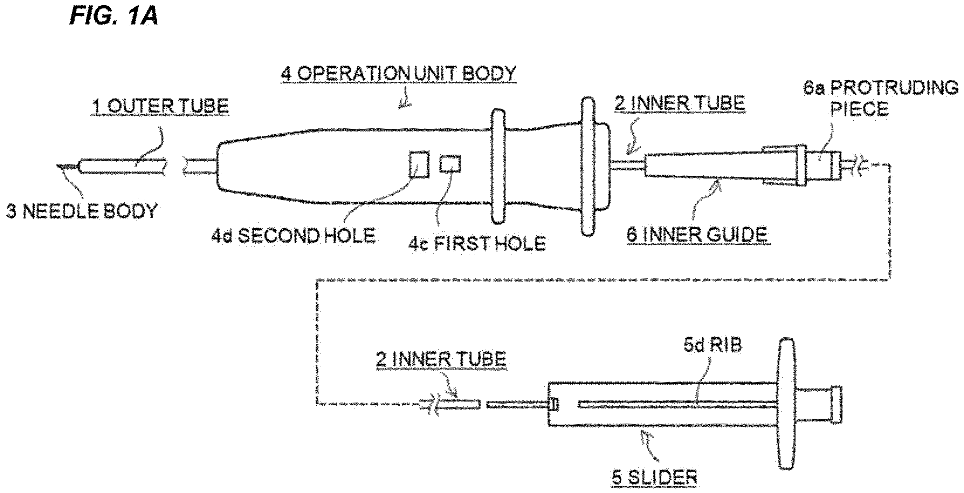

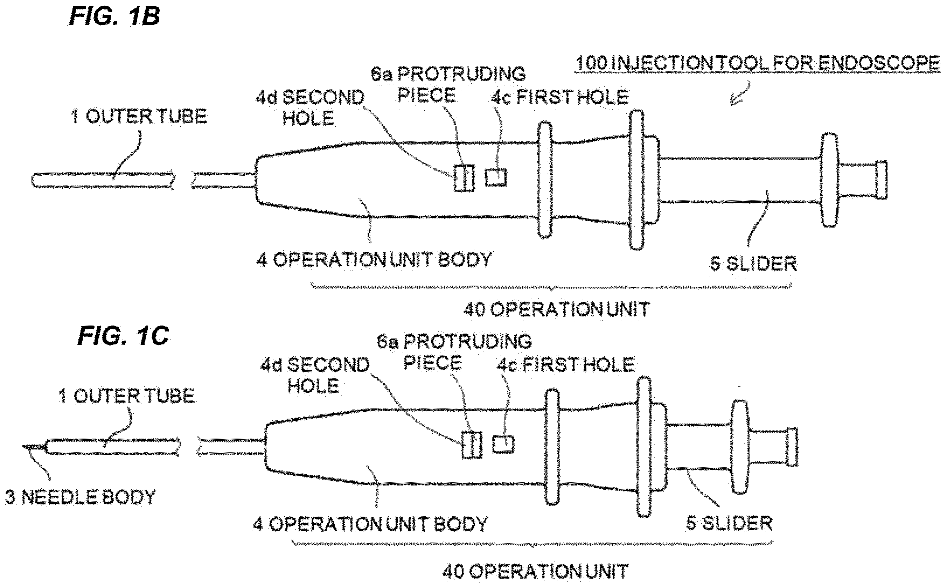

An injection tool 100 for an endoscope according to a first embodiment of the present invention is provided for being inserted into a channel of the endoscope (not shown) and injecting fluid to a submucosal layer within a body cavity. As shown in Fig. 1A representing an exploded and assembled state, the injection tool 100 includes a cylindrical needle body 3 which is inserted into the channel of the endoscope and is provided for injecting a drug solution to an affected area in a state in which the needle body is protruded out of a distal end of the channel of the endoscope, an elongated cylindrical inner tube 2 to which the needle body 3 is attached to a distal end thereof and which is provided for circulating the drug solution from the proximal end side, an elongated cylindrical outer tube 1 which passes the inner tube 2 through an internal tube thereof, an elongated hollow operation unit body 4 which is attached to the proximal end side of the outer tube 1 and has a lumen passing through the inner tube 2 to which the needle body 3 is attached, an inner guide 6 which is inserted into an inner portion of the operation unit body 4 in a state in which the inner tube 2 passes through the inner guide 6, and a slider 5 to which the proximal end side of the inner tube 2 is attached and which moves forward and backward with respect to the operation unit body 4. The injection tool 100 is assembled as shown in Fig. 1B by inserting the inner guide 6 into the operation unit body 4 toward the distal end side, the operation unit body 4 having the distal end to which the outer tube 1 is attached, thereafter inserting the inner tube 2 having the needle body 3 in the distal end so as to insert into the outer tube 1, and inserting the slider 5 into the operation unit body 4.

The injection tool 100 for the endoscope according to the present embodiment is constructed by the operation unit body 4 and the slider 5, and is structured, as shown in Fig. 1C, such that the needle body 3 in the distal end of the inner tube 2 attached to the distal end side of the slider 5 projects and sets with respect to the distal end of the outer tube 1, by relatively moving forward and backward the slider 5 with respect to the operation unit body 4 in a state in which the inner guide 6 and the slider 5 are inserted into the inner portion of the operation unit body 4, as shown in Fig. 1B.

In the present embodiment, a description will be given on the assumption that a left side in the drawing is a distal end side and a right side in the drawing is a proximal end side.

### [Structure of each of parts]

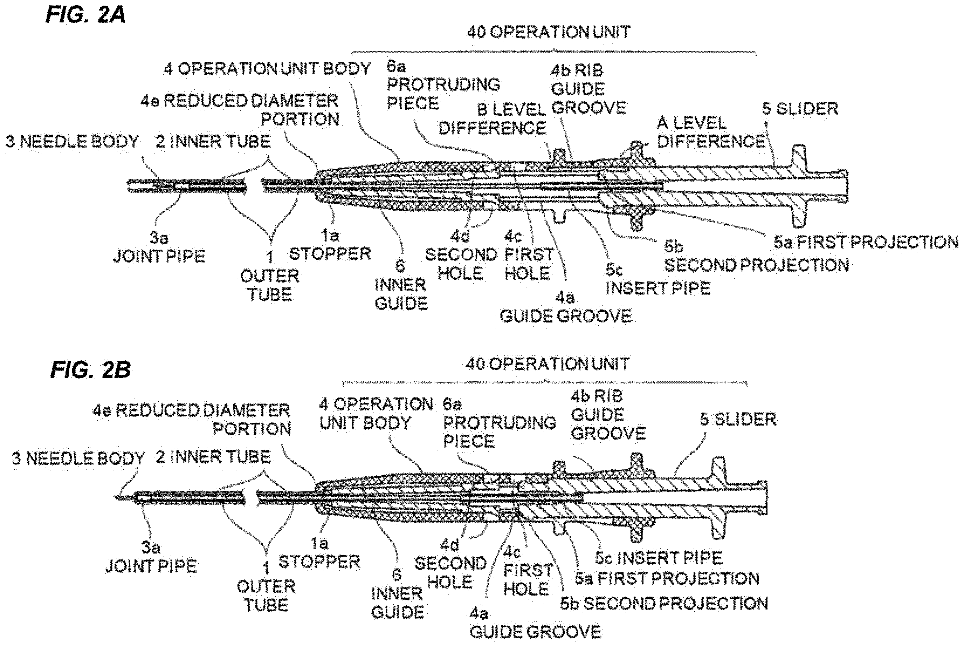

The outer tube 1 is formed into a flexible hollow elongated shape for inserting the inner tube 2 into the inner portion thereof as shown in Figs. 2A and 2B showing a cross section of a state in which the injection tool for the endoscope in Figs. 1B and 1C are turned 45 degrees, and the operation unit body 4 described later is coupled to the proximal end side. Further, the distal end diameter of the outer tube 1 is diameter reduced to an inner side, and a joint pipe 3a attached to the needle body 3 mentioned later gets caught on the inner side, so that the inner tube 2 is configured not to protrude out of the distal end diameter of the outer tube 1.

Further, the outer tube 1 has in the proximal end side a stopper 1a having a diameter increased to an outer peripheral direction, for attaching to the operation unit body 4 described later.

The inner tube 2 is inserted so as to move forward and backward within the outer tube 1 and is formed into a flexible hollow elongated shape for delivering the drug solution as shown in Figs. 2A and 2B. Further, the needle body 3 described later is coupled to the distal end of the inner tube 2 and the slider 5 described later is coupled to the proximal end of the inner tube.

The outer tube 1 and the inner tube 2 are preferable made, for example, of polyether ether ketone (PEEK) and polytetrafluoroethylene (PTFE).

The needle body 3 is formed into a tubular shape and is formed into an inclinedly cut needle shape in the distal end portion, as shown in Figs. 2A and 2B.

An outer periphery of the needle body 3 is attached to a distal end side inner periphery of the inner tube 2 via the cylindrical joint pipe 3a, and is set such that an inner diameter of the inner tube 2 is secured to be larger than an outer diameter of the needle body 3. The needle body 3 may be attached to the inner tube 2 by pressing the needle body 3 to the inner tube 2 without using the joint pipe 3a. A material of the needle body 3 can employ, for example, a hard metal material such as a stainless steel or a hard plastic material.

The operation unit 40 is constructed by the elongated hollow operation unit body 4 which has the lumen passing through the inner tube as mentioned above, and the slider 5 which is retained to the lumen of the operation unit body 4 so as to be slidable in the longitudinal direction.

The slider 5 is formed into a hollow shape as shown in Figs. 2A and 2B, the proximal end side of the slider forms an injection port which can inject the fluid such as the normal saline and the drug solution by connecting a syringe barrel (not shown) thereto, and an insert pipe 5c for connecting the inner tube 2 is attached to the distal end side.

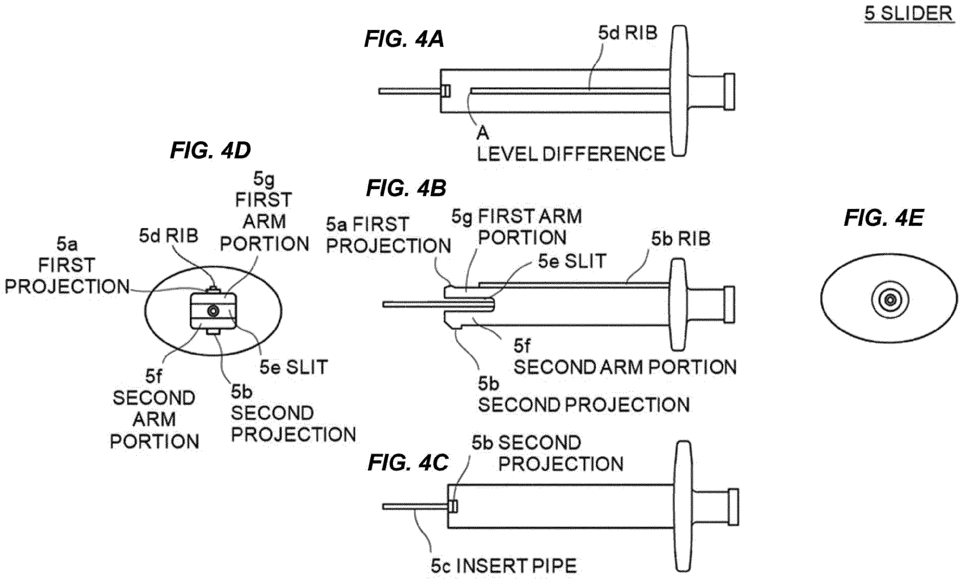

Further, the slider 5 has two arm portions 5g and 5f which are formed at peripherally symmetrical positions in a distal end thereof by a slit 5e formed as a groove and are elastically deformed, and is provided with a first projection 5a which protrudes out of an outer peripheral distal end of the first arm portion 5g for generating a click mating sound mentioned later, a second projection 5b which protrudes out of an outer peripheral distal end of the second arm portion 5f for preventing the slider 5 from easily slipping out of the operation unit body 4, and a rectangular parallelepiped rib 5d which is formed longitudinally closer to the proximal end side of the first projection 5a and a long side of which is positioned in a longitudinal direction for guiding an insertion direction of the slider, as shown in Figs. 4A to 4C representing from a top surface to a bottom surface.

Further, the two arm portions including the first arm portion 5g and the second arm portion 5f are formed in the distal end side of the slider 5 by the slit 5e. Thus, the first projection 5a acts as a leaf spring and is bent inward when coming into contact with the inner wall of the operation unit body 4, and is configured to be expanded at a position of a first hole 4c of the operation unit body 4 mentioned later, thereby having a level difference A with a diameter reduced outer periphery in the distal end of the rib 5d.

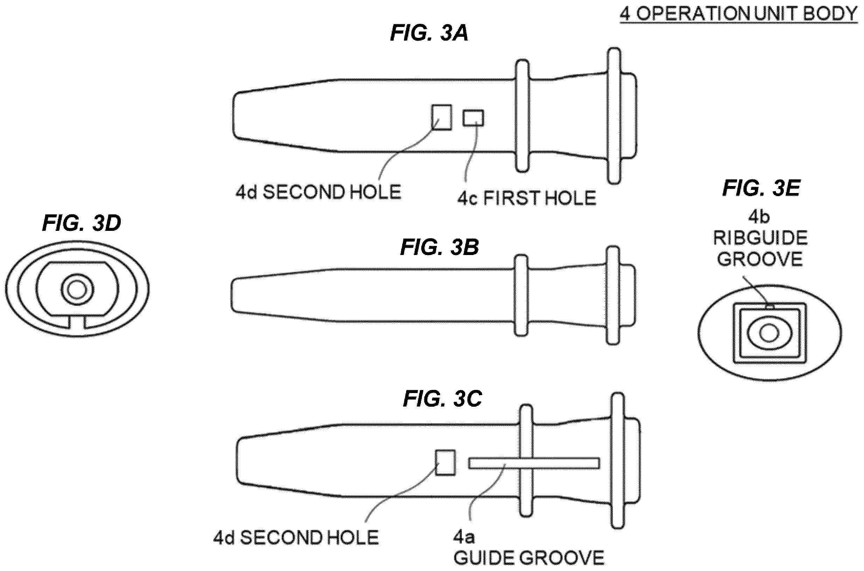

The operation unit body 4 shown in Figs. 3A to 3E is formed into a hollow shape, and has a guide groove 4a to which the second projection 5b of the slider 5 shown in Figs. 2A and 2B is fitted and which is opened so as to allow movement for a longitudinal predetermined distance, and a rib guide groove 4b which is formed longitudinally on an inner periphery of the operation unit body 4. The distal end side of the rib guide groove 4b forms a level difference B (refer to Fig. 2A) in which a diameter of an inner periphery is reduced.

The guide groove 4a has the following function as shown in Fig. 2B. The second projection 5b of the slider 5 hits against the proximal end of the guide groove 4a and the guide groove 4a gets caught on the second projection 5b, thereby preventing the slider 5 from slipping out of the operation body 4, and the second projection 5b of the slider 5 hits against the distal end of the guide groove 4a, so that the guide groove 4a prevents the slider 5 from moving further to the distal end side.

The needle body 3 is housed in the inner portion of the outer tube 1 in a state in which the second projection of the slider 5 is positioned in the proximal end of the guide groove 4a.

Further, at the opposed positions in an outer peripheral direction of the guide groove 4a, there are provided the rib guide groove 4b for longitudinally guiding the rib 5d disposed in the slider 5, and the first hole 4c opened at a position where the first projection 5a of the slider 5 is disposed when the needle body 3 is in a protruded state from the outer tube, closer to the distal end side than the rib guide groove 4b. The first projection 5a of the slider 5 pushes the slider 5 toward the distal end with respect to the operation unit body 4, and mates to the first hole 4c on the basis of sideward expansion of the first arms portion 5g and the second arm portion 5f when the first projection 5a reaches the position of the first hole 4c, thereby generating the click mating sound.

Further, when the level difference A in the distal end of the rib 5d of the slider 5 hits against the level difference B in the distal end of the rib guide groove 4b of the operation unit body 4, the slider 5 is prevented from moving to the distal end side from the hitting position.

The first projection 5a is formed into a mountain shape which is inclined in the distal end side and the proximal end side. On the basis of the mountain shape, an inclined surface of the mountain shape hits against the proximal end side of the first hole 4c so as to prevent the mating from being cancelled when the operator loses hold of the operation unit 40. When the operator intends to detach the first projection 5a from the first hole 4c, the operator strongly pulls the slider 5 toward a rear end with respect to the operation unit body 4, so that the first projection 5a is pushed along the incline of the mountain shape and falls in the operation unit body 4, thereby easily cancelling the mating.

Further, a second hole 4d for fixing the inner guide 6 mentioned later is opened closer to the distal end side than the first hole 4c of the operation unit body 4. The second hole 4d is formed so that a length of a side perpendicular to the longitudinal direction is longer than the first hole 4c.

Further, a reduced diameter portion 4e having a diameter reduced in an inner peripheral direction is formed in the distal end of the operation unit body 4, and the outer tube 1 can be attached to the operation unit body 4 by pulling and hanging the stopper 1a of the outer tube 1 on the reduced diameter portion 4e from the inner side of the operation unit body 4.

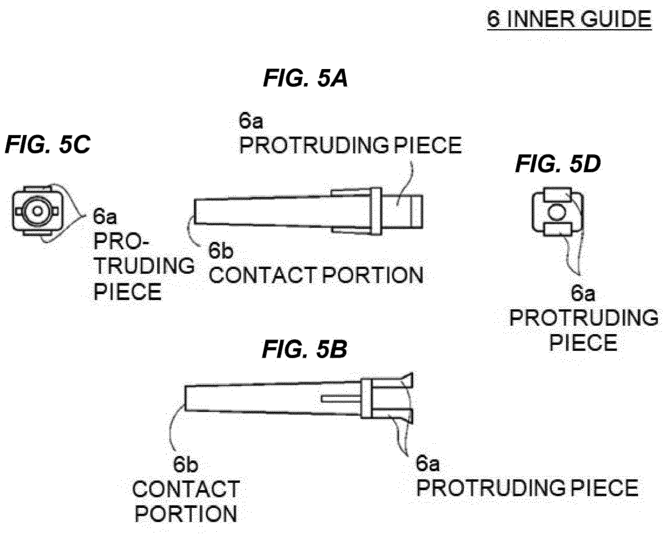

Further, the inner guide 6 shown in Figs. 5A to 5D is fitted to the internal distal end side of the operation unit body 4.

The inner guide 6 is formed into a hollow shape so as to allow the inner tube 2 to be inserted into the inner portion (refer to Figs. 2A and 2B), has in a proximal end side protruding pieces 6a and 6a which extend like an arm shape and expand outward for fitting to the second hole 4d of the operation unit body 4, and has in a distal end side a contact portion 6b. The protruding pieces 6a and 6a are formed so that a length of a side perpendicular to the longitudinal direction of the operation unit body 4 is longer than a side in the same direction of the first hole 4c and the guide groove 4a, for preventing the inner guide 6 from erroneously fitting to the first hole 4c or the guide groove 4a when the inner guide 6 is inserted into the operation unit body 4.

The protruding pieces 6a and 6a are fitted to the second holes 4d and 4d of the operation unit body 4, and the inner guide 6 is then fixed. At this time, the stopper 1aof the outer tube 1 is pressed to the inner side of the reduced diameter portion 4e in the distal end side of the operation unit body 4 by the contact portion 6b in the distal end of the inner guide 6, and is then fixed.

The attachment of the inner guide 6 to the operation unit body 4 is performed by pressing from the proximal end side of the operation unit body 4 toward the distal end side, and the protruding piece 6a acts as the leaf spring and is bent inward when coming into contact with the inner wall of the operation unit body 4, thereby being expanded and fixed at the position of the second hole 4d.

### [Description of motion]

The injection tool 100 for the endoscope according to the present embodiment can project and set the needle body 3 attached to the distal end of the inner tube 2 with respect to the distal end of the outer tube 1 by operating the slider 5 to move forward and backward with respect to the operation unit body 4, as shown in Figs. 1B to 2B. When the slider 5 is pushed forward with respect to the operation unit body 4, the first projection 5a of the slider 5 fits to the first hole 4c of the operation unit body and the clock mating sound is accordingly generated, so that the protrusion of the needle body 3 out of the distal end of the outer tube 1 can be confirmed and the protruded state of the needle body 3 can be kept. Further, the level difference A in the distal end of the rib 5d of the slider 5 hits against the level difference B in the distal end of the rib guide groove 4b of the operation unit body 4, and the second projection 5b of the slider 5 hits against the distal end of the guide groove 4a of the operation unit body 4, thereby preventing the slider 5 from moving toward the distal end side from the position.

When the inward compression of the first projection 5a is cancelled by the fitting when the first projection 5a fits to the first hole 4c of the operation unit body, and the arm portion 5g of the first projection 5a hits against the inner wall of the operation unit 40, the mating sound is generated.

When using the injection tool 100 for the endoscope having the structure mentioned above, the operator inserts into the channel of the endoscope (not shown) which is previously inserted into a body cavity of a patient in a state in which the needle body 3 is housed in the inner portion of the outer tube 1, and protrudes the distal end side out of the distal end of the endoscope near the affected area. Further, the operator taps the needle body 3 to the affected area after protruding the needle body 3 attached to the distal end of the inner tube 2 out of the distal end of the outer tube 1 by operating the slider 5 to move forward and backward with respect to the operation unit body 4. In this state, the operator injects the fluid such as the normal saline and the drug solution to the submucosal layer in the affected area via the inner tube 2 and the needle body 3 from the syringe barrel connected to the slider 5 so as to make the submucosal layer in a floated state, and removes the affected area by using a high frequency knife or a high frequency snare.

### [Effect of embodiments]

The injection tool 100 for the endoscope according to the present embodiment is provided with the first hole 4c in the operation unit body 4 and provided with the first projection 5a in the slider 5. As a result, the first projection 5a of the slider 5 fits to the first hole 4c of the operation unit body by pushing forward the slider 5 with respect to the operation unit body 4, thereby generating the clock mating sound. Thus, the protrusion of the needle body 3 out of the distal end of the outer tube 1 can be easily confirmed, and the protruded state of the needle body 3 can be kept.

Further, according to the present injection tool 100 for the endoscope, in addition to the effect mentioned above, the slider 5 can be prevented from moving further toward the distal end by the contact of the level difference A in the rib 5d of the slider 5 with the level difference B in the rib guide groove 4b of the operation unit body 4, and the contact of the second projection 5b of the slider 5 with the distal end of the guide groove 4a of the operation unit body 4.

Further, according to the present injection tool 100 for the endoscope, the second projection 5b of the slider 5 comes into contact with the proximal end of the guide groove 4a of the operation unit body 4 at the position where the needle body 3 is housed, thereby preventing the slider 5 from slipping out of the operation unit body 4.

Further, according to the present injection tool 100 for the endoscope, the stopper 1a of the outer tube 1 is pulled and hanged on the reduced diameter portion 4e in the distal end of the operation unit body 4, and the stopper 1a of the outer tube 1 is pressed and fixed to the reduced diameter portion 4e of the operation unit body 4 toward the distal end by using the inner guide 6 disposed in the inner portion of the operation unit body 4. Thus, the outer tube 1 can be attached to the operation unit body 4 without using any adhesive agent, and the present invention is in no danger of deterioration of the adhesive agent and is safe.

### REFERENCE SIGNS LIST

- 1: outer tube

- 2: inner tube
- 3: needle body
- 4: operation unit body
- 4a: guide groove
- 4b: rib guide groove
- 4c: first hole
- 4d: second hole
- 5: slider
- 5a: first projection
- 5b: second projection
- 5g: first arm portion
- 5f: second arm portion
- 6: inner guide
- 40: operation unit
- 100: injection tool for endoscope

## Claims

1. An injection tool for an endoscope comprising:
an elongated hollow inner tube for allowing the fluid to flow into;
a hollow needle body provided in a distal end side of the inner tube;
an elongated hollow outer tube into which the inner tube is insertable to be movable forward and backward in a longitudinal direction;
an operation unit having an elongated hollow operation unit body with a lumen attached to a proximal end side of the outer tube and passing through the inner tube, and a slider having the inner tube attached thereto, inserted into the lumen of the operation unit body and moved forward and backward in the longitudinal direction; and
an inner guide inserted from the proximal end side of the operation unit body,
the injection tool for the endoscope allowing the needle body to project and set with respect to the distal end of the outer tube by operating the slider of the operation unit to move forward and backward in the longitudinal direction,
wherein the slider has in a distal end thereof a first projection protruding in an outer peripheral direction with an elastic force,
wherein the operation unit body has a first hole open to the distal end side in such a manner that the first projection in the distal end of the slider fits thereto when the needle body in the protruded state from the outer tube,
wherein the outer tube has in a proximal end thereof a stopper expanded to an outer peripheral direction in such a manner as to lock to a reduced diameter portion of the operation unit body when the outer tube is inserted from the proximal end side of the operation unit body, and
wherein the inner guide has a contact portion bringing the stopper of the outer tube into contact with the reduced diameter portion of the operation unit body so as to fix.

2. The injection tool for the endoscope according to claim 1, wherein the first projection is formed into a mountain shape which is fitted with a slope to the first hole opened to the operation unit body.

3. The injection tool for the endoscope according to claim 1 or 2, wherein the slider has a second projection protruding toward the outer peripheral direction, and
wherein the operation unit body has at a symmetrical position of the first hole in the outer peripheral direction a guide groove to which the second projection is fitted to allow the operation unit body to move at a predetermined distance in the longitudinal direction.

4. The injection tool for the endoscope according to claim 3, wherein the slider has a first arm portion having in a distal end thereof the first projection, and a second arm portion having in a distal end thereof the second projection.

5. The injection tool for the endoscope according to claim 4, wherein the inner guide has in the proximal end thereof a protruding piece protruding in the outer peripheral direction, and
wherein the operation unit body has closer to the distal end side than the first hole a second hole to which the protruding piece fits.

6. The injection tool for the endoscope according to claim 1 or 2, wherein the slider has a rectangular parallelepiped rib having a long side in a longitudinal direction for guiding an insertion direction of the slider, and
wherein the operation unit body has in a longitudinal direction a rib guide groove to which the rib is fitted.

7. The injection tool for the endoscope according to claim 3, wherein the slider has a rectangular parallelepiped rib having a long side in a longitudinal direction for guiding an insertion direction of the slider, and
wherein the operation unit body has in a longitudinal direction a rib guide groove to which the rib is fitted.

8. The injection tool for the endoscope according to claim 4, wherein the slider has a rectangular parallelepiped rib having a long side in a longitudinal direction for guiding an insertion direction of the slider, and
wherein the operation unit body has in a longitudinal direction a rib guide groove to which the rib is fitted.

9. The injection tool for the endoscope according to claim 7, wherein the slider has a rectangular parallelepiped rib having a long side in a longitudinal direction for guiding an insertion direction of the slider, and
wherein the operation unit body has in a longitudinal direction a rib guide groove to which the rib is fitted.

10. The injection tool for the endoscope according to claim 8, wherein the slider has a rectangular parallelepiped rib having a long side in a longitudinal direction for guiding an insertion direction of the slider, and
wherein the operation unit body has in a longitudinal direction a rib guide groove to which the rib is fitted.
